# FASCICULE DE BREVET EUROPEEN

(11) **EP 1 531 729 B1**
(45) Date de publication et mention de la délivrance du brevet: **29.08.2012**
(21) Numéro de dépôt: 03756017.4
(22) Date de dépôt: 28.05.2003
(51) Int. Cl.: A61B 7/02, B06B 1/00, G10K 11/26, G10K 11/34, A61B 8/15

(54) **PROCEDE POUR GENERER UN CHAMP D ONDES PREDETERMINE**
VERFAHREN ZUM ERZEUGEN EINES VORBESTIMMTEN WELLENFELDES
METHOD OF GENERATING A PREDETERMINED WAVE FIELD

(30) Priorité: 04.06.2002 FR 0206846
(43) Date de publication de la demande: 25.05.2005
(73) Titulaire: SUPER SONIC IMAGINE, 13857 Aix-en-Provence (FR)
(72) Inventeur: MONTALDO, Gabriel, F-75017 Paris (FR); FINK, Mathias, F-92190 Meudon (FR); TANTER, Mickael, F-75006 Paris (FR)
(74) Mandataire: Burbaud, Eric
(86) Numéro de dépôt international: PCT/FR2003/001617
(87) Numéro de publication internationale: WO 2003/101302

(56) Documents cités:
- FR-A- 2 700 878
- FR-A- 2 815 717
- US-A- 5 207 214
- CASSEREAU D ET AL: "Theoretical and experimental analysis of focusing techniques through liquid-solid interfaces" ULTRASONICS SYMPOSIUM, 1994. PROCEEDINGS., 1994 IEEE CANNES, FRANCE 1-4 NOV. 1994, NEW YORK, NY, USA,IEEE, US, 1 novembre 1994 (1994-11-01), pages 1075-1080, XP010139694 ISBN: 0-7803-2012-3
- CHAKROUN N ET AL: "Ultrasonic nondestructive testing with time reversal mirrors" ULTRASONICS SYMPOSIUM, 1992. PROCEEDINGS., IEEE 1992 TUCSON, AZ, USA 20-23 OCT. 1992, NEW YORK, NY, USA,IEEE, US, 20 octobre 1992 (1992-10-20), pages 809-814, XP010103667 ISBN: 0-7803-0562-0

## Description

La présente invention est relative aux procédés pour générer des champs d'ondes prédéterminés dans un milieu.

Le champ d'ondes en question peut consister en une impulsion d'onde focalisée en un ou plusieurs points du milieu, ou il peut s'agir d'un champ spatio-temporel plus complexe.

Plus particulièrement, l'invention concerne un procédé pour générer un champ d'ondes objectif prédéterminé dans un milieu (homogène ou hétérogène) au moyen d'un premier réseau comprenant au moins un transducteur et un deuxième réseau comprenant au moins un transducteur, ce procédé comprenant une étape d'apprentissage au cours de laquelle on détermine, en transmettant des ondes dans le milieu entre le premier réseau et le deuxième réseau (le deuxième réseau peut éventuellement comprendre des transducteurs communs avec le premier réseau), des signaux ei(t) à émettre par chaque transducteur i du premier réseau pour générer ledit champ d'ondes objectif prédéterminé dans le milieu, ledit champ d'ondes objectif prédéterminé correspondant à un signal objectif oj(t) pour chaque transducteur j du deuxième réseau.

Le document WO-A-02/32316 décrit un exemple d'un tel procédé, dans lequel l'étape d'apprentissage susmentionnée permet de déterminer des signaux à appliquer aux transducteurs du premier réseau pour focaliser une impulsion d'ondes respectivement sur chaque transducteur du deuxième réseau, ce qui permet ensuite de déterminer comment focaliser des impulsions d'ondes en d'autres points du milieu pour imager ce milieu par ondes ultrasons. Ce procédé connu donne toute satisfaction au plan de ses résultats, mais nécessite toutefois des moyens de calcul importants et implique en outre des temps de calcul assez longs au cours de l'étape d'apprentissage.

La présente invention a notamment pour but de pallier ces inconvénients.

A cet effet, selon l'invention, un procédé du genre en question est caractérisé en ce que l'étape d'apprentissage comprend la séquence de correction suivante :
(a) faire émettre simultanément par chaque transducteur i du premier réseau un signal ei(t) déterminé par avance (ces signaux ei(t) peuvent initialement être prédéterminés ou précédemment déterminés par voie expérimentale comme décrit ci-après, ou ces signaux peuvent résulter de l'étape (g) ci-dessous d'une itération antérieure de la séquence de correction) et destinés à générer un champ d'ondes réel correspondant au champ d'ondes objectif prédéterminé dans le milieu, ,
(b) faire capter par chaque transducteur j du deuxième réseau un signal rj (t) résultant du champ d'ondes généré par les signaux ei(t),
(c) déterminer un signal de différence temporellement inversé dj(-t), pour chaque transducteur j du deuxième réseau, dj(-t) étant l'inversion temporelle de la différence dj(t)=rj(t)-oj(t),
(d) faire émettre simultanément le signal de différence temporellement inversé dj(-t) par chaque transducteur j du deuxième réseau,
(e) faire capter un signal c'i(t) par chaque transducteur i du premier réseau à partir des ondes générées par les signaux de différence temporellement inversés dj(-t),
(f) déterminer un signal de correction ci(t)=β.c'i(-t) pour chaque transducteur i du premier réseau, c'i(-t) étant l'inversion temporelle du signal capté c'i(t) et β étant un nombre réel positif non nul choisi de façon que β<(∥*e̅*∥.∥*d̅*∥)/∥*r̅*∥.∥*c̅*∥), où *e̅* = [*ei*(*t*)], *d̅* = [*dj*(*t*)], *r̅*=[*rj*(*t*)]*,* *̅c̅*̅'̅=[*c'i*(*t*)] et ∥ ∥ désigne une norme vectorielle,
(g) corriger le signal ei(t) en lui soustrayant ci (t) .

Grâce à ces dispositions, on parvient à générer très précisément le champ d'ondes objectif, après une ou plusieurs itérations de la séquence de correction et ce même dans un milieu de propagation très dissipatif et/ou hétérogène.

Dans des modes de réalisation préférés de l'invention, on peut éventuellement avoir recours en outre à l'une et/ou à l'autre des dispositions suivantes :
- la séquence de correction est répétée plusieurs fois ;
- la séquence de correction est précédée d'une étape initiale au cours de laquelle on détermine expérimentalement une première valeur du signal ei(t) pour chaque transducteur i du premier réseau ;
- au cours de l'étape initiale, on détermine l'inversion temporelle oj(-t) du signal objectif pour chaque transducteur du deuxième réseau, on fait émettre ladite inversion temporelle oj(-t) du signal objectif par chaque transducteur j du deuxième réseau, on fait capter par chaque transducteur i du premier réseau un signal e'i(t) résultant du champ d'ondes généré par les signaux oj(-t), et on détermine le signal ei(t)=e'i(-t) pour chaque transducteur du premier réseau, e'i(-t) étant l'inversion temporelle du signal e'i(t) ;
- la norme vectorielle est définie comme suit : ∥*x̅*∥[*xₘ*(*t*)]∥= *Max*(|*xₘ*(*t*)|), où |*xₘ*(*t*)| désigne l'amplitude du signal xₘ(t) ;
- le champ d'ondes est un champ d'ondes acoustiques ;
- le champ d'ondes est un champ d'ondes électromagnétiques ;
- les ondes sont générées par un système de télécommunication.

D'autres caractéristiques et avantages de l'invention apparaîtront au cours de la description suivante d'une de ses formes de réalisation, donnée à titre d'exemple non limitatif, en regard du dessin joint.

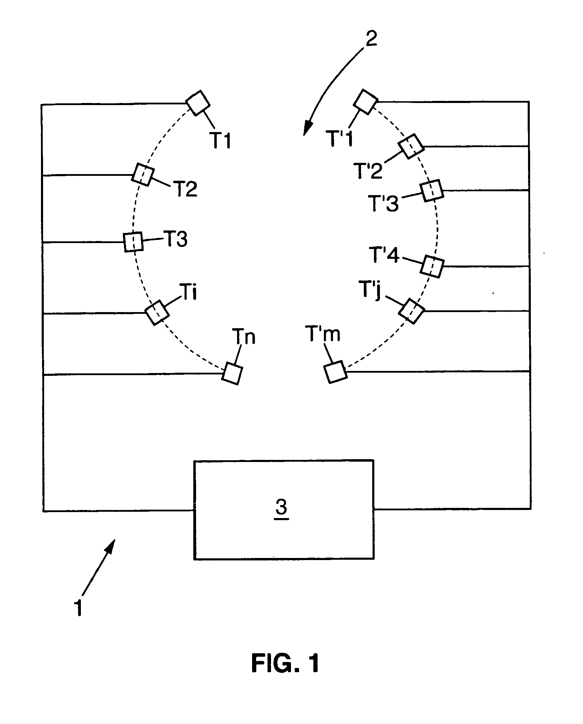

Sur le dessin, la figure 1 est un schéma de principe représentant un exemple de dispositif permettant de mettre en oeuvre l'invention.

Le dispositif 1 de génération d'ondes représenté sur le dessin peut être notamment :
- un dispositif de génération d'ondes acoustiques, auquel cas il peut s'agir par exemple d'un dispositif d'imagerie ultrasonore, d'un dispositif de sonorisation, d'un dispositif anti-bruit actif, d'un dispositif de thérapie ultrasonore (par exemple, lithotritie), ou d'un dispositif de communication, notamment sous-marine, par ondes acoustiques,
- ou, le cas échéant, un dispositif de génération d'ondes électromagnétiques, auquel cas il peut s'agir d'un dispositif de télécommunications.

Le dispositif 1 est destiné à générer des ondes dans un milieu 2, qui suivant le cas, peut être :
- une partie d'un corps humain.ou animal à imager ou à traiter (imagerie médicale ultrasonore ou thérapie ultrasonore),
- une partie d'un objet à imager (imagerie industrielle ultrasonore),
- le milieu sous-marin ou souterrain (télécommunications par voie acoustique),
- un lieu public ou privé (sonorisation ou système anti-bruit actif),
- une partie de la surface terrestre avec les couches basses correspondantes de l'atmosphère (télécommunications radio entre des bases fixes et des mobiles),
- la surface terrestre et l'atmosphère y compris ses couches hautes (télécommunications terrestres à longue distance par voie radio ou télécommunications radio entre la terre et un ou plusieurs satellites), etc.

Dans les différentes applications susmentionnées, il est nécessaire de pouvoir générer avec le plus de précision possible un ou plusieurs champs d'ondes objectifs prédéterminés dans le milieu 2, par exemple pour pouvoir focaliser les ondes émises par un premier réseau de transducteurs T1, T2 ... Tn en un ou plusieurs points du milieu 2 ou le cas échéant pour générer des champs d'ondes plus complexes.

L'intérêt de pouvoir effectuer une focalisation de grande précision peut être par exemple de réaliser une image d'une partie du milieu 2 avec une grande précision, ou de détruire sélectivement une partie du milieu 2 (thérapie ultrasonore), et encore d'envoyer un ou plusieurs messages à des endroits spécifiques du milieu et non dans le reste du milieu 2 (soit dans un souci de discrétion, soit dans un souci d'éviter les interférences entre les différents messages et de permettre ainsi une augmentation du débit de télécommunications).

Le premier réseau comprend un nombre n au moins égal à 1 (avantageusement au moins égal à 2) de transducteurs T1-Tn capables d'émettre et de recevoir des ondes, par exemple ultrasonores.

Les signaux ei(t) qui doivent être émis par les transducteurs Ti pour obtenir le ou les champs d'ondes objectifs prédéterminés, sont obtenus au cours d'une étape d'apprentissage, au cours de laquelle un deuxième réseau de transducteurs T'l-T'm est utilisé.

Ce deuxième réseau comprend un nombre n au moins égal à 1 (avantageusement au moins égal 2) de transducteurs T'l-T'm de même type que les transducteurs Tl-Tn.

Ce deuxième réseau peut être distinct du premier réseau T1-Tn, et n'être mis en place dans le milieu 2 qu'au cours de l'étape d'apprentissage, puis enlevé.

Il serait toutefois possible de concevoir de mettre en oeuvre le procédé de la présente invention avec un ensemble de transducteurs restant en place en permanence dans le milieu, certains de ces transducteurs servant à constituer le premier réseau de transducteurs et d'autres de ces transducteurs servant à constituer le second réseau de transducteurs pendant la phase d'apprentissage. Au moins certains transducteurs pourraient d'ailleurs être communs aux premier et deuxième réseaux ou encore appartenir soit au premier réseau, soit au deuxième réseau suivant le champ d'ondes objectif que l'on cherche à obtenir (et notamment suivant le point du milieu 2 sur lequel on cherche à focaliser les ondes émises).

Les différents transducteurs T1-Tn, T'1-T'm sont commandés par un dispositif de commande électronique 3 qui ne sera pas décrit en détails ici. Ce dispositif de commande peut par exemple être identique ou similaire au dispositif de commande décrit dans le document WO-A-02/32316 susmentionné lorsque le dispositif 1 est un dispositif d'imagerie ou de thérapie acoustique ultrasonore.

Les signaux ei(t) déterminés au cours de l'étape d'apprentissage pour chaque transducteur Ti du premier réseau permettent par exemple de générer dans le milieu 2 un champ d'ondes focalisé uniquement en un point où est situé l'un des transducteurs du deuxième réseau, par exemple le transducteur T'1.

Cette étape d'apprentissage peut bien entendu être renouvelée pour chacun des transducteurs T'1-T'm du deuxième réseau, de façon à déterminer à chaque fois des signaux ei(t) permettant de focaliser le champ d'ondes sur l'un quelconque des points où est situé l'un des transducteurs T'j du deuxième réseau.

Dans tous les cas de figure, au cours d'une même étape d'apprentissage, on détermine les signaux ei(t) qui doivent être émis par les transducteurs Ti du premier réseau pour obtenir des signaux objectifs oj(t) correspondant au champ d'ondes objectif au niveau de chaque transducteur T'j du deuxième réseau.

Le dispositif de commande 3 peut éventuellement avoir en mémoire, à l'avance, des valeurs initiales des signaux ei(t) permettant d'obtenir approximativement le champ d'ondes souhaité.

Toutefois, dans un mode de réalisation préféré de l'invention, ces valeurs initiales des signaux ei(t) sont déterminées au cours d'une étape initiale dans laquelle :
- on fait émettre simultanément par les transducteurs Tj du deuxième réseau, des signaux oj(-t), résultant de l'inversion temporelle des signaux objectifs oj (t) (dans le cas où les signaux objectifs oj (t) consistent soit en de simples impulsions à t=0, soit en des signaux plats, cette étape revient simplement à faire émettre les signaux objectifs oj(t) par les transducteurs T'j),
- on fait capter par les transducteurs Ti du premier réseau des signaux e'i(t) résultant du champ d'ondes généré par les signaux oj(-t),
- et on détermine la valeur initiale ei(t) par inversion temporelle des signaux e'i(t) susmentionnés : ei(t)=e'i(-t).

Une fois déterminée la valeur initiale du signal ei(t) pour chaque transducteur Ti du premier réseau, on procède à une ou plusieurs itérations de la séquence de correction suivantes :
(a) on fait émettre simultanément par les différents transducteurs Ti du premier réseau les signaux ei(t),
(b) on fait capter, par les différents transducteurs T'j du deuxième réseau, des signaux rj(t) résultant du champ d'ondes généré par les signaux ei(t),
(c) on détermine un signal de différence temporellement inversé dj (-t) pour chaque transducteur j du deuxième réseau, dj(-t) étant l'inversion temporelle de la différence dj (t)=rj (t)-oj (t),
(d) on fait émettre simultanément le signal de différence temporellement inversé dj(-t) par chaque transducteur j du deuxième réseau,
(e) on fait capter un signal c'i(t) par chaque transducteur i du premier réseau à partir des ondes générées par les signaux de différence temporellement inversés dj(-t),
(f) on détermine un signal de correction ci(t)=β.c'i(-t) pour chaque transducteur i du premier réseau, c'i(-t) étant l'inversion temporelle du signal capté c'i(t) et β étant un nombre réel positif non nul choisi de façon que β<(∥*e̅*∥.∥*d̅*∥)/∥*r̅*∥.∥*c̅'̅*∥), où *e̅*=[*ei*(*t*)], *d̅*=[*dj*(*t*)], *r̅*=[*rj*(*t*)], *c̅*'=[*c'i*(*t*)] et ∥∥ désigne une norme vectorielle (par exemple telle que ∥*x̅*∥=∥[*x̅ₘ*]| = *Max*(|*xₘ*|), où |*xₘ*(*t*)| désigne l'amplitude du signal xₘ(t)). La valeur de β définie ci-dessus (généralement supérieure à 1) permet au processus de correction de converger très rapidement vers des signaux ei(t) répondant à l'objectif recherché, mais le coefficient β pourrait également être pris égal à 1 sans pour autant sortir du cadre de l'invention,
(g) corriger le signal ei(t) en lui soustrayant ci(t).

A l'itération suivante de la séquence de correction, la valeur du signal ei(t) utilisée à l'étape (a) est ensuite celle précédemment déterminée à l'étape (g) de la séquence de correction décrite ci-dessus.

L'expérience montre que le processus de correction converge très rapidement, en quelques millisecondes, même dans un milieu très dissipatif et/ou hétérogène.

Cette convergence rapide, qui ne nécessite par ailleurs pas de moyens de calcul lourds, permet le cas échéant au système de s'adapter en temps réel à des modifications du milieu lorsque le milieu est changeant, ce qui est notamment le cas dans les applications de télécommunications par voie radio ou par voie acoustique. Dans ce cas, le deuxième réseau de transducteurs ne sera pas enlevé après la ou les étapes d'apprentissage de départ, mais sera au contraire laissé en place de façon à pouvoir réitérer la ou les étapes d'apprentissage, à intervalles de temps réguliers ou non.

On notera que dans tout le processus d'apprentissage explicité ci-dessus, les signaux émis sont donnés à des coefficients multiplicatifs constants (non nuls) près.

Une fois la ou les étapes d'apprentissage terminées le dispositif de génération d'ondes 1 est capable de générer un ou plusieurs champs d'ondes prédéterminés dans le milieu 2 avec une très grande précision.

Par exemple, dans le cas où on aurait procédé à plusieurs étapes d'apprentissage permettant de générer avec précision une impulsion localisée uniquement en un point occupé par un transducteur T'j du deuxième réseau, on peut ensuite :
- dans les applications d'imagerie, générer des impulsions localisées en des points quelconques du milieu 2 (en générant des signaux Ei(t) obtenus soit par des procédés simples d'interpolation entre les signaux d'émission ei(t)ⱼ permettant de focaliser les ondes respectivement sur plusieurs transducteurs j du deuxième réseau, soit par des procédés plus complexes tels que ceux décrits par exemple dans le document WO-A-02/32316 susmentionné),
- dans les applications de thérapie ultrasonore, générer une impulsion d'ondes de grande amplitude en un point particulier du milieu destiné à être détruit, ce point particulier pouvant correspondre soit à l'emplacement d'un des transducteurs T'j du deuxième réseau, soit à un point différent du milieu 2, auquel cas les signaux permettant de générer cette impulsion sont déterminés comme expliqué au paragraphe précédent,
- dans les applications de télécommunications, générer un signal porteur d'informations en un point du milieu 2 (comme aux deux alinéas précédents, ce point du milieu 2 peut être l'un des points occupés par les transducteurs du deuxième réseau ou un autre point du milieu, auquel cas on détermine les signaux à émettre Ei(t) par interpolation ou par des procédés plus complexes tels que ceux décrits dans le document WO-A-02/32316 à partir des différents signaux ei(t)ⱼ permettant de focaliser, les ondes sur les points occupés par les transducteurs Tj du deuxième réseau), ce signal porteur d'informations étant obtenu en faisant émettre par les transducteurs Ti des signaux Si(t)=Ei(t)⊗S(t) égaux au produit de convolution des signaux Ei(t), avec le signal S(t) porteur d'informations qui doit être transmis au point voulu.

## Revendications

1. Procédé pour générer un champ d'ondes objectif prédéterminé dans un milieu au moyen d'un premier réseau comprenant au moins un transducteur et un deuxième réseau comprenant au moins un transducteur, ce procédé comprenant une étape d'apprentissage au cours de laquelle on détermine, en transmettant des ondes dans le milieu entre le premier réseau et le deuxième réseau, des signaux ei(t) à émettre par chaque transducteur i du premier réseau pour générer ledit champ d'ondes objectif prédéterminé dans le milieu, ledit champ d'ondes objectif prédéterminé correspondant à un signal objectif oj(t) pour chaque transducteur j du deuxième réseau
**caractérisé en ce que** l'étape d'apprentissage comprend la séquence de correction suivante :
(a) faire émettre simultanément par chaque transducteur i du premier réseau un signal ei(t) déterminé par avance et destiné à générer un champ d'ondes réel correspondant au champ d'ondes objectif prédéterminé dans le milieu,
(b) faire capter par chaque transducteur j du deuxième réseau un signal rj(t) résultant du champ d'ondes généré par les signaux ei(t),
(c) déterminer un signal de différence temporellement inversé dj(-t) pour chaque transducteur j du deuxième réseau, dj(-t) étant l'inversion temporelle de la différence dj(t)=rj(t)-oj(t),
(d) faire émettre simultanément le signal de différence temporellement inversé dj(-t) par chaque transducteur j du deuxième réseau,
(e) faire capter un signal c'i(t) par chaque transducteur i du premier réseau à partir des ondes générées par les signaux de différence temporellement inversés dj(-t),
(f) déterminer un signal de correction ci(t)=β.c'i(-t) pour chaque transducteur i du premier réseau, c'i(-t) étant l'inversion temporelle du signal capté c'i(t) et β étant un nombre réel positif non nul choisi de façon que β<(∥*e̅*∥.∥*d̅*∥)/∥*r̅*∥.∥*c̅*∥), où *e̅*=[*ei*(*t*)], *d̅*=[*dj*(*t*)], *r̅*=[*rj*(*t*)], *c̅*'=[*c'i*(*t*)] et ∥∥ désigne une norme vectorielle,
(g) corriger le signal ei(t) en lui soustrayant ci(t).

2. Procédé selon la revendication 1, dans lequel la séquence de correction est répétée plusieurs fois.

3. Procédé selon l'une quelconque des revendications précédentes, dans lequel la séquence de correction est précédée d'une étape initiale au cours de laquelle on détermine expérimentalement une première valeur du signal ei(t) pour chaque transducteur i du premier réseau.

4. Procédé selon la revendication 3, dans lequel, au cours de l'étape initiale :
- on détermine l'inversion temporelle oj(-t) du signal objectif pour chaque transducteur du deuxième réseau,
- on fait émettre ladite inversion temporelle oj(-t) du signal objectif par chaque transducteur j du deuxième réseau,
- on fait capter par chaque transducteur i du premier réseau un signal e'i(t) résultant du champ d'ondes généré par les signaux oj(-t),
- et on détermine le signal ei(t)=e'i(-t) pour chaque transducteur du premier réseau, e'i(-t) étant l'inversion temporelle du signal e'i(t).

5. Procédé selon l'une quelconque des revendications précédentes, dans lequel la norme vectorielle est définie comme suit : ∥*x̅*∥=∥[*xₘ*]|=*Max(*|*xₘ*|), où |*xₘ*(*t*)| désigne l'amplitude du signal xₘ(t).

6. Procédé selon l'une quelconque des revendications précédentes, dans lequel le champ d'ondes est un champ d'ondes acoustiques.

7. Procédé selon l'une quelconque des revendications 1 à 5, dans lequel le champ d'ondes est un champ d'ondes électromagnétiques.

8. Procédé selon l'une quelconque des revendications précédentes, dans lequel les ondes sont générées par un système de télécommunication.

## Claims

1. A method for generating a predetermined objective wave field in a medium by means of a first network comprising at least one transducer and a second network comprising at least one transducer, this method comprising a learning step in the course of which signals ei(t) to be emitted by each transducer i of the first network so as to generate said predetermined objective wave field in the medium are determined by transmitting waves in the medium between the first network and the second network, said objective wave field corresponding to an objective signal oj(t) for each transducer j of the second network,
**characterized in that** the learning step comprises the following correction sequence:
(a) making each transducer i of the first network simultaneously emit a signal ei(t) determined in advance and intended for generating a real wave field corresponding to the predetermined objective wave field in the medium,
(b) making each transducer j of the second network capture a signal rj(t) resulting from the wave field generated by the signals ei(t),
(c) determining a time reversed difference signal dj(-t) for each transducer j of the second network, dj(-t) being the time reversal of the difference dj(t)=rj(t)-oj(t),
(d) making each transducer j of the second network simultaneously emit the time reversed difference signal dj(-t),
(e) making each transducer i of the first network capture a signal c'i(t) based on the waves generated by the time reversed difference signals dj(-t),
(f) determining a correction signal ci(t)=β.c'i(-t) for each transducer i of the first network, c'i(-t) being the time reversal of the captured signal c'i(t) and β being a positive nonzero real number chosen in such a way that β<(∥*e̅*∥.∥*d̅*∥)/(∥*r̅*∥.∥*c̅'*∥) where *e̅*=[*ei*(*t*)], *d̅*=[*dj*(*t*)], *r̅*=[*rj*(*t*)], *c̅*'=[*c'i*(*t*)] and ∥∥ designates a vector norm,
(g) correcting the signal ei(t) by subtracting ci(t) therefrom.

2. The method as claimed in claim 1, wherein the correction sequence is repeated several times.

3. The method as claimed in any one of the preceding claims, wherein the correction sequence is preceded by an initial step in the course of which a first value of the signal ei(t) is determined experimentally for each transducer i of the first network.

4. The method as claimed in claim 3, wherein, in the course of the initial step:
- the time reversal oj(-t) of the objective signal is determined for each transducer of the second network,
- each transducer j of the second network is made to emit said time reversal oj(-t) of the objective signal,
- each transducer i of the first network is made to capture a signal e'i(t) resulting from the wave field generated by the signals oj(-t),
- and the signal ei(t)=e'i(-t) is determined for each transducer of the first network, e'i(-t) being the time reversal of the signal e'i(t).

5. The method as claimed in any one of the preceding claims, wherein the vector norm is defined as follows:
∥*x̅*∥=∥[*xₘ*]∥=*Max*(|*xₘ*|), where |*xₘ*(*t*)| designates the amplitude of the signal xₘ(t).

6. The method as claimed in any one of the preceding claims, wherein the wave field is an acoustic wave field.

7. The method as claimed in any one of claims 1 to 5, wherein the wave field is an electromagnetic wave field.

8. The method as claimed in any one of the preceding claims, wherein the waves are generated by a telecommunication system.

## Patentansprüche

1. Verfahren zum Erzeugen eines vorgegebenen objektives Wellenfelds in einer Umgebung mit Hilfe eines erstes Netzes, umfassend mindestens einen Wandler, und ein zweites Netz, umfassend mindestens einen Wandler, wobei dieses Verfahren einen Schritt des Lernprozesses umfasst, während dessen Wellen in die Umgebung zwischen dem ersten Netz und dem zweiten Netz übertragen werden und damit Signale ei(t) festgelegt werden, die von jedem Wandler i des ersten Netzes ausgesandt werden sollen, um das vorgegebene objektive Wellenfeld in der Umgebung zu erzeugen, wobei das vorgegebene objektive Wellenfeld einem objektiven Signal oj(t) für jeden Wandler j des zweiten Netzes entspricht,
**dadurch gekennzeichnet, dass** der Schritt des Lernprozesses die folgende Korrekturabfolge umfasst:
(a) gleichzeitig von jedem Wandler i des ersten Netzes vorab ein bestimmtes Signal ei(t) aussenden lassen, das ein aktuelles Wellenfeld erzeugen soll, dass dem vorgegebenen objektives Wellenfeld in der Umgebung entspricht,
(b) von jedem Wandler j des zweiten Netzes ein Signal rj(t) empfangen lassen, das von dem Wellenfeld herrührt, das durch die Signale ei(t) erzeugt wird,
(c) ein zeitweise umgekehrtes Differenzsignal dj(-t) für jeden Wandler j des zweiten Netzes festlegen, wobei dj(-t) die Zeitumkehr der Differenz dj(t)= rj(t)-oj(t) ist.
(d) gleichzeitig das zeitliche umgekehrte Differenzsignal (-t) von jedem Wandler j des zweiten Netzes aussenden lassen,
(e) von jedem Wandler i des ersten Netzes anhand von Wellen, die durch die zeitlich umgekehrten Differenzsignale dj(-t) erzeugt werden, ein Signal c'i(t) empfangen lassen,
(f) ein Korrektursignal ci(t)=β.c'i(-t) für jeden Wandler i des ersten Netzes festlegen, wobei c'i(-t) die Zeitumkehr des empfangenen Signals c'i(t) ist und β eine positive reelle Zahl ungleich Null ist, die so gewählt ist, dass β<(∥*e̅*∥.∥*d̅*∥)/∥*r̅*∥.∥*c̅'*∥), wobei *e̅*=[*ei*(*t*)], *d̅*=[*dj*(*t*)], *r̅*=[*rj*(*t*)], *c̅'*=[*c'i*(*t*)] und ∥∥ eine Vektornorm bezeichnet,
(g) das Signal ei(t) korrigieren, indem ci(t) subtrahiert wird.

2. Verfahren nach Anspruch 1, wobei die Korrekturabfolge mehrmals wiederholt wird.

3. Verfahren nach einem der vorhergehenden Ansprüche, wobei der Korrekturabfolge ein erster Schritt vorausgeht, während dessen experimentell ein erster Wert des Signals ei(t) für jeden Wandler i des ersten Netzes bestimmt wird.

4. Verfahren nach Anspruch 3, wobei während des erstens Schritts:
- die Zeitumkehr oj(-t) des objektiven Signals für jeden Wandler des zweiten Netzes festgelegt wird,
- die Zeitumkehr oj(-t) des objektiven Signals für jeden Wandler j des zweiten Netzes ausgesandt wird,
- von jedem Wandler i des ersten Netzes ein Signal e'(t) empfangen wird, das sich aus dem Wellenfeld ergibt, das von den Signalen oj(-t) erzeugt wird,
- das Signal ei(t)=e'i(-t) für jeden Wandler des ersten Netzes bestimmt wird, wobei e'i(t) die Zeitumkehr des Signals e'i(t) ist.

5. Verfahren nach einem der vorhergehenden Ansprüche, wobei die Vektornorm wie folgt festgelegt wird:
∥*x̅*∥=|[*xₘ*]|=*Max*(|*xₘ*|), wobei |xₘ(t)| die Signalamplitude |xₘ(t)| bezeichnet.

6. Verfahren nach einem der vorhergehenden Ansprüche, wobei das Wellenfeld ein akustisches Feld ist.

7. Verfahren nach einem der Ansprüche 1 bis 5, wobei das Wellenfeld ein elektromagnetisches Feld ist.

8. Verfahren nach einem der vorhergehenden Ansprüche, wobei die Wellen durch ein Telekommunikationssystem erzeugt werden.
